# EUROPÄISCHE PATENTSCHRIFT

(11) **EP 1 033 111 B1**
(45) Veröffentlichungstag und Bekanntmachung des Hinweises auf die Patenterteilung: **06.05.2004**
(21) Anmeldenummer: 00810083.6
(22) Anmeldetag: 31.01.2000
(51) Int. Cl.: A61B 17/72, A61B 17/74

(54) **Knochenschraube zur Verankerung eines Marknagels**
Bone screw for the anchoring of an intramedullary nail
Vis à os pour l'ancrage d'un clou médullaire

(30) Priorität: 01.03.1999 EP 99810171
(43) Veröffentlichungstag der Anmeldung: 06.09.2000
(73) Patentinhaber: Centerpulse Orthopedics Ltd., 6340 Baar (CH)
(72) Erfinder: Adam, Michael, 6330 Cham (CH)
(74) Vertreter: Manitz, Finsterwald & Partner Gbr

(56) Entgegenhaltungen:
- EP-A- 0 424 734
- EP-A- 0 865 769
- US-A- 4 622 959
- US-A- 5 536 127
- US-A- 5 713 901

## Beschreibung

Die Erfindung handelt von einer Knochenschraube zur Verankerung eines Marknagels, welche Schraube mit einem Kopfteil für das Ansetzen eines Eindrehwerkzeuges versehen ist und den Marknagel in einer passenden Querbohrung mit einem distalen Teil quer durchdringt und mit einem Mittelteil abstützt, wobei der Mittelteil und der distale Teil ein Gewinde mit einer Steigung S und einem Kerndurchmesser D₁ und einem Aussendurchmesser D₃ aufweisen.

Marknägel werden zur Stützung von Frakturen an Röhrenknochen angewendet. In der Regel werden Marknägel in den Markhohlraum eingeschlagen, um dem Knochen eine Ausrichtung in Längsrichtung zu geben und um anschliessend mit schraubenähnlichen Verankerungselementen, die durch Querbohrungen im Marknagel geführt sind, eine zusätzliche Verankerung des Marknagels im Röhrenknochen zu bewirken.

In der Patentschrift US 4,622,959 ist ein Marknagel gezeigt, der nach seinem Einschlagen im Femurknochen an seinem proximalen Ende mit einer Zieleinrichtung verbunden wird, um fluchtend zu seinen bestehenden Querbohrungen den Röhrenknochen vorzubohren und anschliessend Verankerungsschrauben einzudrehen, die sich beidseitig vom Marknagel mit ihrem Gewinde abstützen.

Verankerungsstifte und -schrauben für Marknägel sind auch in der Offenlegungsschrift DE-A-32 44 243 sowie bei dem deutschen Gebrauchsmuster G 89 07443.2 gezeigt. Den aufgeführten Referenzen ist gemeinsam, dass die Fixierung der Knochenschraube in ihrem proximalen Bereich mit dem gleichen Aussendurchmesser, wie er beim lateralen Gewindeteil vorliegt, erfolgt. Die Fixierung erfolgt dabei in der relativ harten Kortikalis des Röhrenknochens. Ein durchgehendes Schraubengewinde, welches mit seinem mittleren Bereich den Marknagel stützen soll, hat den Nachteil, dass der Knochen im proximalen Bereich der Schraube wegen der grossen Einschraublänge ausleiert. Eine zylindrische Fortsetzung des lateralen Schraubengewindes mit seinem Aussendurchmesser in den proximalen Bereich ergibt eine Sprengwirkung im Knochen, der nur durch eine Annäherung des Kerndurchmessers an den Aussendurchmesser des Gewindes begegnet werden kann. Das heisst, das Gewinde im lateralen Bereich der Schraube ist in einem solchen Fall im proximalen Bereich durch die Sprengwirkung des zylindrischen Teils in seiner Formgebung beschränkt.

Aufgabe der vorliegenden Erfindung ist es, die Fixierung einer solchen Sicherungsschraube zu verbessern. Dies wird mit dem Kennzeichen vom unabhängigen Anspruch 1 erreicht, indem das Gewinde einen flachen zylindrischen Gewindegrund mit einer Länge I > 0,3 S besitzt und indem im proximalen Kopfteil ein weiteres Gewinde mit gleichem Kerndurchmesser D₁ und mit gleicher Gewinde mit gleichem Kerndurchmesser D₁ und mit gleicher Steigung S wie im distalen Teil, jedoch mit einem grösseren äusseren Durchmesser D₂ > D₃ angebracht ist, um proximal und distal eine gute Fixierung zu erreichen.

Ein Vorteil dieser Anordnung besteht darin, dass die Geometrie der beiden Gewinde an die geringe Nachgiebigkeit der Kortikalis anpassbar ist. Beide Gewinde legen während ihrem gemeinsamen Eindringen in die Kortikalis einen kurzen Weg mit einem jeweils eigenen Gewindeprofil zurück.

Die abhängigen Ansprüche 1 bis 9 stellen vorteilhafte Weiterbildungen der Erfindung dar.

Mit einem Aussendurchmesser des proximalen Gewindes, der grösser als der Innendurchmesser der Querbohrung im Marknagel ist, wird sichergestellt, dass sich der proximale Teil ein eigenes Gewinde im Knochen formt. Das Gewindeprofil ist dazu asymmetrisch ausgebildet mit einer Neigung gegen proximal, die grösser als die Neigung gegen distal ist, um die spezifische Druckbelastung, die auf der gegen proximal gerichteten Flanke zur Erzeugung einer Vorschubkraft auftritt, klein zu halten. Neigungen des vorstehenden Gewindeprofils gegen distal zwischen 40° und 50° und gegen proximal zwischen 75° und 87° sind vorgesehen.

Durch die Gewindeprofile sind Kerben gelegt, sodass Schneiden entstehen, die während dem Einschrauben übermässige Radialkräfte und ein Sprengen des Knochens verhindern.

Weiterhin ist es vorteilhaft, die Gewindespitzen des Gewindeprofils im Mittelteil und im distalen Teil mit einer Abflachung 0,1 mm ≤ a ≤ 0,8 zu versehen, damit eine ausreichend tragende Fläche zwischen Querbohrung und Knochenschraube vorhanden ist. Eine weitere Verbesserung lässt sich erzielen, wenn die rückseitig gegen proximal gerichteten Flanken auf einer gemeinsamen Wendel liegen, weil dann unabhängig von der momentanen Einschraubetiefe eine geführte Vorschubbewegung vorhanden ist.

Für die Herstellung der beiden Gewindeteile mit gemeinsamer Wendel für die gegen proximal gerichtete Flanke und auch allgemein für zwei unterschiedliche Gewindeprofile ist Zirkularfräsen mit zwei entsprechenden Erzeugenden sinnvoll. Die Profilfräser sind mit ihrer Achse jeweils zur Schraubenachse verschwenkt und werden relativ zum Schraubenrohling wendelförmig herumgeführt, um den Gewindegrund zu fräsen.

Weiterhin ist es vorteilhaft im Bereich proximal des Gewindes am Kopfteil einen Einstich oder eine Ausnehmung anzubringen, die verhindert, dass der Kerndurchmesser des Gewindes konisch ausläuft. Gerade an dieser Stelle kann eine nicht gewollte Sprengwirkung auftreten, welche Risse im Knochen erzeugt.

Im folgenden ist die Erfindung anhand von Ausführungsbeispielen beschrieben. Es zeigen:
- Fig. 1: Schematisch einen Längsschnitt durch einen Femurknochen mit einem Marknagel, welcher an Querbohrungen mit Knochenschrauben gesichert ist;
- Fig. 2: schematisch eine vergrösserte Ansicht einer passenden Knochenschraube; und
- Fig. 3: schematisch einen vergrösserten Ausschnitt einer weiteren Knochenschraube, welche durch Zirkularfräsen herstellbar ist.

In den Figuren sind Knochenschrauben 1 gezeigt zur Verankerung eines Marknagels 2 mit Querbohrungen in einem Röhrenknochen. Die Knochenschrauben 1 besitzen ein Gewinde 7 mit Steigung S, mit Kerndurchmesser D₁ und mit Aussendurchmesser D₃, welches mit dem mittleren Teil 5 der Schraube den Marknagel stützt und mit dem distalen Teil 6 die Schraube 1 im Knochen fixiert, wobei das Gewinde einen flachen zylindrischen Gewindegrund 9 mit einer Länge I > 0,3 S aufweist. Ausserdem besteht im proximalen Teil 3 ein zweites Gewinde 7a mit gleicher Steigung S und mit Kerndurchmesser D1, jedoch mit einem grösseren äusseren Durchmesser D₂ > D₃, um proximal mit einem grösseren Gewindeprofil eine Fixierung zu erreichen.

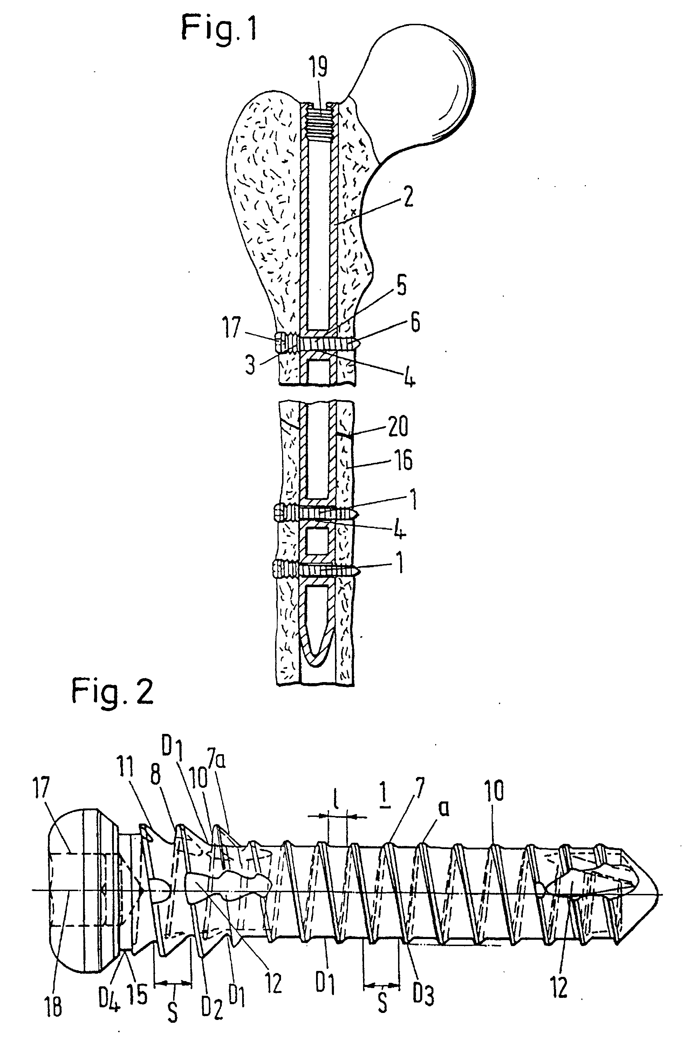

In Fig. 1 überbrückt ein Marknagel 2 eine Bruchstelle 20 in einem Femurknochen 16. Beidseits der Bruchstelle 20 besitzt der Marknagel 2 Querbohrungen 4, welche Knochenschrauben 1 führen, die ihrerseits distal und proximal mit unterschiedlich grossen Gewindeprofilen 7, 7a gleicher Steigung S im Knochen 16 fixiert sind. Mit einer am Marknagel 2 mit einer Befestigung 19 anschliessbaren Zielvorrichtung (hier nicht gezeigt) werden zu den Querbohrungen 4 konzentrische Bohrungen in den Knochen 16 vorgebohrt, um anschliessend die Knochenschrauben 1 mit Hilfe von einem Innensechskant 17 des proximalen Kopfteils 3 einzudrehen.

Eine erste Knochenschraube 1 ist in Fig. 2 gezeigt. Ihr Kopfteil 3 beginnt proximal mit einem eigentlichen Schraubenkopf, welcher einen Innensechskant 17 und ein grosses Gewindeprofil 7a mit Kerndurchmesser D₁ und Aussendurchmesser D₂ und mit einer Steigung S besitzt, welches durch Kerben 12 unterbrochen ist. Die rückseitige, gegen proximal gerichtete Flanke 8 des Gewindeprofils 7a bildet mit der Achse 18 der Schraube eine Neigung 10 von 83° und die gegen distal gerichtete Flanke eine Neigung 11 von 45°. Das Gewinde 7a endet gegen proximal in einer Nut 15, welche das Gewinde 7a vom eigentlichen Schraubenkopf trennt. Der Innendurchmesser D₄ der Nut 15 ist deutlich kleiner als der Aussendurchmesser D₂ des Gewindes 7a. Im mittleren und distalen Teil der Schraube 1 ist ein zweites Gewinde 7 ebenfalls mit der Steigung S und dem Kerndurchmesser D₁ angebracht. Dieses Gewinde 7 liegt mit seiner gegen proximal gerichteten Flanke auf der gleichen Wendel wie die gegen proximal gerichtete Flanke 8 des Gewindes 7a am Kopfteil. Hingegen ist der Aussendurchmesser D₃ des zweiten Gewindes 7 deutlich kleiner, sodass unter Beibehaltung der Neigungen 10, 11 ein kleineres Gewindeprofil 7 mit einem zylindrisch flachen Gewindegrund einer Länge I > 0,3 S entsteht. Eine Ausführungsvariante sieht zum Beispiel eine Steigung S = 1,75 mm, einen Kerndurchmesser D₁ = 4,3 mm, einen Aussendurchmesser D₂ = 6,5 mm im proximalen Gewinde 7a und einen Aussendurchmesser D₃ = 4,9 mm im mittleren und distalen Gewindeteil vor, während eine Abflachung a = 0,2 mm für die Gewindespitzen vorgesehen ist. Im distalen Teil sind zur Schraubenspitze hin ebenfalls Kerben vorgesehen, die Schneiden entstehen lassen, um zu grosse Ringspannungen im Knochen zu verhindern.

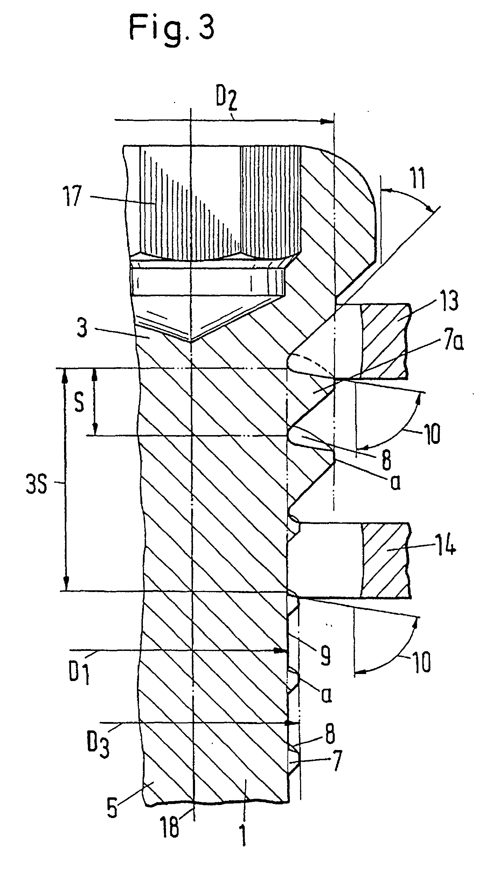

In Fig. 3 ist ein weiteres Beispiel mit gleichen Hinweiszeichen wie in Fig. 2 gezeigt. Zusätzlich sind Profilfräser 13, 14 gezeigt, welche beim Zirkularfräsen nacheinander zum Einsatz kommen, um die beiden Gewinde 7a, 7 mit einer durchgehenden Wendel der gegen proximal gerichteten Flanke 8 herzustellen. Nachdem der Schraubenrohling 3 mit seinen Aussendurchmessern D₂, D₃ vorbearbeitet wurde, wird zunächst im proximalen Kopfteil 3 ein Gewinde 7a mit einem Profilfräser 13 gefräst. Der entsprechend der Steigung S schräggestellte Profilfräser 13 wird zunächst radial zur Schraubenachse 18 abgesenkt und anschliessend auf einer Schraubenlinie mit der Steigung S an der Schraube 1 entlang bewegt, um das Gewinde 7a vom Kopfteil 3 zu fräsen. Anschliessend wird ein zweiter Profilfräser 14 in einem Abstand zum Profil des ersten Fräsers 13 in Eingriff gebracht, um das zweite Gewinde 7 zu fräsen, wobei der Abstand der gegen proximal gerichteten Flanken 8 einem ganzzahligen Vielfachen der Steigung S im gleichen Längsschnitt entspricht.

## Patentansprüche

1. Knochenschraube (1) zur Verankerung eines Marknagels (2), welche Schraube mit einem Kopfteil (3) für das Ansetzen eines Eindrehwerkzeuges versehen ist und den Marknagel (2) in einer passenden Querbohrung (4) mit einem distalen Teil (6) quer durchdringt und mit einem Mittelteil (5) abstützt, wobei der Mittelteil und der distale Teil ein Gewinde (7) mit einer Steigung S und einem Kerndurchmesser D₁ und einem Aussendurchmesser D₃ aufweisen, **dadurch gekennzeichnet, dass** das Gewinde (7) einen flachen zylindrischen Gewindegrund mit einer Länge I > 0,3 S besitzt und dass im proximalen Kopfteil (3) ein weiteres Gewinde (7a) mit gleichem Kerndurchmesser D₁ und mit gleicher Gewinde (7a) mit gleichem Kerndurchmesser D₁ und mit gleicher Steigung S wie im distalen Teil (6), jedoch mit einem grösseren äusseren Durchmesser D₂ > D₃ angebracht ist, um proximal und distal eine gute Fixierung zu erreichen.

2. Knochenschraube nach Anspruch 1, **dadurch gekennzeichnet, dass** der Aussendurchmesser D2 des proximalen Gewindes (7a) grösser als der Innendurchmesser der Querbohrung (4) im Marknagel ist.

3. Knochenschraube nach Anspruch 1, **dadurch gekennzeichnet, dass** das proximal vorstehende Gewindeprofil asymmetrisch ausgebildet ist mit einer Neigung (10) gegen proximal, die grösser ist als die Neigung (11) gegen distal.

4. Knochenschraube nach Anspruch 3, **dadurch gekennzeichnet, dass** die Neigung des vorstehenden Gewindeprofils zwischen 40° und 50° gegen distal (11) und zwischen 75° und 87° gegen proximal (10) beträgt.

5. Knochenschraube nach einem der Ansprüche 1 bis 3, **dadurch gekennzeichnet, dass** durch die Gewindeprofile Kerben (12) gelegt sind, die ein Schneiden des Gewindeprofils beim Einschrauben ermöglichen.

6. Knochenschraube nach einem der Ansprüche 1 bis 4, **dadurch gekennzeichnet, dass** die Gewindespitzen des Gewindeprofils im Mittelteil (5) und im distalen Teil (6) eine Abflachung 0,1 mm ≤ a ≤ 0,8 mm aufweisen.

7. Knochenschraube nach einem der Ansprüche 1 bis 5, **dadurch gekennzeichnet, dass** die rückseitige, gegen proximal gerichtete Flanke (8) für beide Gewinde (7, 7a) auf einer gemeinsamen Wendel liegt.

8. Knochenschraube nach einem der Ansprüche 1 bis 6, **dadurch gekennzeichnet, dass** im Bereich proximal des Gewindes (7a) am Kopfteil (3) eine Ausnehmung (15) mit einem Durchmesser D₄ < D2 angebracht ist, um einen unzulässigen Anstieg des Kerndurchmessers beim Einschrauben zu verhindern.

9. Verfahren zur Herstellung einer Knochenschraube nach einem der Ansprüche 1 bis 8, **dadurch gekennzeichnet, dass** die Gewindeprofile vom Kopfteil (3) und von Mittelteil (5) und distalem Teil (6) mit Profilfräsern (13, 14) durch Zirkularfräsen erzeugt werden, um zwei unterschiedliche Gewindeprofile mit gleicher Steigung S zu erzeugen.

## Claims

1. Bone screw (1) for anchoring a medullary nail (2), said screw being provided with a head part (3) for the application of a turning-in tool and penetrating the medullary nail (2) transversely with a distal part (6) in a suitable transverse bore (4) and being supported with a middle part (5), with the middle part and the distal part having a thread (7) with a pitch S and a core diameter D₁ and an outer diameter D₃, **characterised in that** the thread (7) has a flat cylindrical thread base with a length 1 > 0.3 S; and **in that**, in the proximal head part (3), a further thread (7a) with the same core diameter D₁ and with similar thread (7a) with the same core diameter D₁ and with the same pitch S as in the distal part (6), however with a greater outer diameter D₂ > D₃, is provided in order to achieve a good fixing proximally and distally.

2. Bone screw in accordance with claim 1 **characterised in that** the outer diameter D₂ of the proximal thread (7a) is greater than the inner diameter of the transverse bore (4) in the medullary nail.

3. Bone screw in accordance with claim 1 **characterised in that** the proximally projecting thread profile is designed asymmetrically with an inclination (10) towards the proximal which is greater than the inclination (11) towards the distal.

4. Bone screw in accordance with claim 3 **characterised in that** the inclination of the projecting thread profile amounts to between 40° and 50° towards the distal (11) and between 75° and 87° towards the proximal (10).

5. Bone screw in accordance with any one of the claims 1 to 3 **characterised in that** notches (12) are laid through the thread profiles which enable a cutting of the thread profile during the screwing in.

6. Bone screw in accordance with any one of the claims 1 to 4 **characterised in that** the thread tips of the thread profile in the middle part (5) and in the distal part (6) have a flattening of 0.1 mm ≤ a ≤ 0.8 mm.

7. Bone screw in accordance with any one of the claims 1 to 5 **characterised in that** the reverse-side, proximally directed flank (8) lies on a helix which is common to both threads (7, 7a).

8. Bone screw in accordance with any one of the claims 1 to 6 **characterised in that**, in the region proximal of the thread (7a) at the head part (3), a cut-out (15) with a diameter D₄ < D₂ is provided in order to prevent an impermissible increase of the core diameter during the screwing in.

9. Method for the manufacture of a bone screw in accordance with any one of the claims 1 to 8, **characterised in that** the thread profiles of the head part (3) and of the middle part (5) and distal part (6) are produced through circular milling with profile milling tools (13, 14) in order to produce two different thread profiles with the same pitch S.

## Revendications

1. Vis à os (1) pour l'ancrage d'un clou médullaire (2), ladite vis étant munie d'une partie tête (3) pour l'application d'un outil de vissage et traversant transversalement le clou médullaire (2) dans un perçage transversal (4) adapté avec une partie (6) distale et prenant appui, par une partie centrale (5), la partie centrale (5) et la partie distale présentant un filetage (7) d'un pas S et d'un diamètre de noyau D₁ et d'un diamètre extérieur D₃, **caractérisée en ce que** le filetage (7) comporte un fond de filetage cylindrique d'une longueur 1 > 0,3 S, et **en ce que**, dans la partie tête (3) proximale, est ménagé un autre filetage (7a) de même diamètre de noyau D₁ et de même pas S que dans la partie la partie distale (6), cependant ayant un diamètre extérieur D₂ > D₃, afin d'obtenir une bonne fixation proximale et distale.

2. Vis à os selon la revendication 1, **caractérisée en ce que** le diamètre extérieur D₂ du filetage proximal (7a) est supérieur au diamètre intérieur du perçage transversal (4) dans le clou médullaire.

3. Vis à os selon la revendication 1, **caractérisée en ce que** le profil de filetage, en saillie en situation proximale, est asymétrique et a une inclinaison (10) vers la partie proximale supérieure à l'inclinaison (11) vers la partie distale.

4. Vis à os selon la revendication 3, **caractérisée en ce que** l'inclinaison du profil de filetage en saillie est comprise entre 40° et 50° vers la partie distale (11) et est comprise entre 75° et 87° vers la partie proximale (10).

5. Vis à os selon l'une des revendications 1 à 3, **caractérisée en ce que**, de par le profil de filetage, sont positionnées des entailles (12) permettant un découpage du profil de taraudage lors du vissage.

6. Vis à os selon l'une des revendications 1 à 4, **caractérisée en ce que** les pointes de filetage du profil fileté, dans la partie médiane (5) et dans la partie distale (6), présentent un méplat de 0,1 mm ≤ a ≤ 0,8 mm.

7. Vis à os selon l'une des revendications 1 à 5, **caractérisée en ce que** le flanc arrière (8), tourné vers la partie proximale, des deux filetages (7, 7a) est situé sur une hélice commune.

8. Vis à os selon l'une des revendications 1 à 6, **caractérisée en ce que** dans la zone proximale du filetage (7a), sur la partie tête (3), est ménagé un évidement (15) d'un diamètre D₄ < D₂, afin d'empêcher tout placement en position oblique inadmissible du diamètre de noyau lors du vissage.

9. Procédé de fabrication d'une vis à os selon l'une des revendications 1 à 8, **caractérisé en ce que** les profils de filetage, de la partie tête (3) et de la partie centrale (5) et de la partie distale (6) sont générés par des fraises de profilage (13, 14), en procédant à un fraisage circulaire pour générer deux profils de filetage différents mais de même pas S.
